# EUROPEAN PATENT SPECIFICATION

(11) **EP 4 092 048 B1**
(45) Date of publication and mention of the grant of the patent: **15.10.2025**
(21) Application number: 21741754.2
(22) Date of filing: 05.01.2021
(51) Int. Cl.: C07K 14/55, C07K 1/107, A61K 38/20, A61K 47/60, A61P 31/04, A61P 31/12, A61P 35/00, A61P 37/02

(54) **PREPARATION METHOD FOR PEGYLATED BIOMOLECULES HAVING CONTROLLABLE BINDING SITES**
HERSTELLUNGSVERFAHREN FÜR PEGYLIERTE BIOMOLEKÜLE MIT KONTROLLIERBAREN BINDUNGSSTELLEN
PROCÉDÉ DE PRÉPARATION DE BIOMOLÉCULES PEGYLÉES AYANT DES SITES DE LIAISON POUVANT ÊTRE CONTRÔLÉS

(30) Priority: 15.01.2020 CN 202010043172
(43) Date of publication of application: 23.11.2022
(73) Proprietor: Jenkem Technology Co., Ltd. (Tianjin), Tianjin 300462 (CN)
(72) Inventor: WANG, Qingbin, Tianjin 300462 (CN); QI, Jie, Tianjin 300462 (CN); LI, Yu, Tianjin 300462 (CN)
(74) Representative: HGF
(86) International application number: PCT/CN2021/070264
(87) International publication number: WO 2021/143572

(56) References cited:
- WO-A1-2019/131964
- WO-A1-2019/226538
- CN-A- 1 453 292
- CN-A- 101 591 649
- CN-A- 103 517 718
- CN-A- 107 106 654
- CN-A- 109 589 415
- CN-A- 111 378 026
- SULTAN HUSSEIN ET AL: "Sustained Persistence of IL2 Signaling Enhances the Antitumor Effect of Peptide Vaccines through T-cell Expansion and Preventing PD-1 Inhibition", vol. 6, no. 5, 30 April 2018 (2018-04-30), US, pages 617 - 627, XP093054719, ISSN: 2326-6066, Retrieved from the Internet <URL:https://aacrjournals.org/cancerimmunolres/article-pdf/6/5/617/2342195/617.pdf> DOI: 10.1158/2326-6066.CIR-17-0549
- WANG YANMING, ZHOU XIN-BO: "An Biased CD122 Receptor Agonist——NKTR-214", CLINICAL MEDICATION JOURNAL, vol. 17, no. 2, 1 February 2019 (2019-02-01), pages 1 - 31, XP055828845, ISSN: 1672-3384, DOI: 10.3969/j.issn.1672-3384.2019.02.001
- WANG XUDONG, LI XIAO-HUI, SU ZHI-GUO, XIU ZHI-LONG: "Site-specific PEGylation Strategies and Suitable Modified Sites of Protein Drugs", CHINA BIOTECHNOLOGY, vol. 30, no. 4, 1 January 2010 (2010-01-01), pages 101 - 109, XP055828846, DOI: 10.13523/j.cb.20100418
- D. H. CHARYCH, U. HOCH, J. L. LANGOWSKI, S. R. LEE, M. K. ADDEPALLI, P. B. KIRK, D. SHENG, X. LIU, P. W. SIMS, L. A. VANDERVEEN, C: "NKTR-214, an Engineered Cytokine with Biased IL2 Receptor Binding, Increased Tumor Exposure, and Marked Efficacy in Mouse Tumor Models", CLINICAL CANCER RESEARCH, AMERICAN ASSOCIATION FOR CANCER RESEARCH, US, vol. 22, no. 3, 1 February 2016 (2016-02-01), US, pages 680 - 690, XP055432446, ISSN: 1078-0432, DOI: 10.1158/1078-0432.CCR-15-1631

## Description

### TECHNICAL FIELD

The present invention relates to the technical field of pharmaceuticals, and particularly to a method for preparing a PEGylated IL-2 .

### BACKGROUND

Interleukin-2 (IL-2) is produced when lymphocyte proliferation is stimulated by a mitogen such as phytohemagglutinin (PHA). Recently, studies on this factor and its receptors are becoming deeper, and it has been found that the factor plays an important role in cellular immunity, humoral immunity and immune regulation. In particular, the role and clinical significance in tumor immunotherapy have been appreciated by many authors.

IL-2 is prominently produced by CD4+ T helper cells (Ths), while a minority is produced by CD8+ and other immune cells. These cells secrete IL-2 under the action of IL-1. IL-2 produced by lymphocytes acts on cells having IL-2 receptors on their surfaces, causing cells to proliferate to exert their biological function. It may act on normal T and B lymphocytes, precursor cells of NK cells, cytotoxic T lymphocytes (CTLs), precursor cells of lymphokine-activated killer cells and the like. The biological functions of IL-2 include: 1, maintaining the long-term survival of activated T cells *in vitro* and maintaining their biological functional activity, and enhancing the function to kill tumor cells of some T cells activated by tumor antigens and thereby enhancing the anti-tumor effect; 2, promoting the proliferation, differentiation and antibody production of B cells; and 3, stimulating the secretion of granulocyte-macrophage colony stimulating factor, γ-interferon and the like, and promoting lymphocytes to express the receptors; maintaining cell proliferation in thymus in the presence of a sub-dose mitogen; and promoting the expression of IL-2 receptors, participating in the immune regulation system of organism, and maintaining immune balance and stability.

IL-2 is the first immunotherapeutic drug. In 1992 and 1998, the U.S. FDA approved an IL-2 drug aldesleukin for treating advanced kidney cancer and malignant melanoma, respectively, showing an efficacy rate of 15%-20% and a prolonged survival more than 5 years in nearly 10%-15% of the patients. However, due to the high clinical doses, IL-2 demonstrated significant side effects, such as severe hypotension and vascular leak syndrome. At that time, patients treated with IL-2 had to be hospitalized and closely observed at all times, and a slightest inadvertence could be life-threatening. Therefore, the use of IL-2 is limited by the great side effects, and most clinicians do not dare to prescribe it easily.

The mechanism of action of IL-2 is as follows: IL-2 is produced mainly by CD4+ T cells stimulated by antigen, and can also be secreted by CD8+ T cells, NK cells and DC cells. A high dose of IL-2 can promote the differentiation of CD4+ T cells and the proliferation of CD8+ T cells and NK cells, and increase lethality of these cells, thereby mobilizing the immune responses of the body. On the contrary, a low dose of IL-2 can promote the proliferation of regulatory T cells and have an inhibitory effect on the immune system, and is thus often used to treat autoimmune diseases.

This difference is mainly due to the different distribution of IL-2 receptors on the surface of immune cells, which are composed of three subunits, i.e., α, β and γ subunits. The receptors on the surface of killer cells such as CD8 positive T cells, NK cells and the like are mainly composed of β and γ subunits (IL-2Rβγ), having low affinity for IL-2 and requiring a high dose of IL-2 to activate. The receptors on the surface of regulatory T cells are mostly antibodies with high affinity composed of three subunits of α, β and γ (IL-2Rαβγ), and can be easily activated by a low dose of IL-2 to exert the immunosuppressive effect.

Though IL-2 was approved for treating renal cell carcinoma and metastatic melanoma more than 20 years ago, it is rarely used because of the following: 1, the sole use of IL-2 gives a low efficacy rate of about 15%-20%; 2, IL-2 has dual functions that a low dose of IL-2 promotes the proliferation of regulatory T cells to cause immunosuppression, leaving a problem of balancing the two functions; 3, the compound features a short half-life is short of only a few minutes, requiring a high-dose administration for maintaining the efficacy; 4, the immune system may be overactivated by the serious adverse reactions, attacking the organs, causing organ failure and leading to a narrow dosage window. In order to improve the efficacy of IL-2 and reduce adverse reactions, scientists have also tried various combination therapies, such as IL-2 + interferon, IL-2 + lymphokine-activated killer (LAK) cell therapy, IL-2 + chemotherapy and the like, but the efficacy was not ideal.

In recent years, scientists have discovered that: adding chemical modifications to the normal IL-2 molecule can maintain the effect of IL-2 while reducing the concentration for use and the side effects. Then, NKTR-214 was developed. NKTR-214 is essentially an upgraded version of an existing IL-2 drug that was launched on the market more than 20 years ago. It has lower side effects and better specificity (more likely to activate CD8 rather than Treg cells), and can activate the immune system more efficiently. Specifically, 6 polyethylene glycol (PEG) modifications are added to the IL-2 molecule to form an inactive drug. Surprisingly, after the drug is injected into patients with tumors, the 6 PEG modifications gradually drop off, forming active 2-PEG and 1-PEG forms.

NKTR-214 is a PEGylated IL-2, and the activity of the prodrug is very low. Six PEG chains are ingeniously linked to the sites in IL-2 that can bind to the α subunit of a high-affinity IL-2Rαβγ antibody, making the PEGylated IL-2 more prone to bind to IL-2Rβγ receptors in killer immune cells. The activity of the drug is slowly restored as PEG chains are degraded. NKTR-214 resolves two major difficulties of IL-2 in tumor treatment: it achieves the sustained drug release through PEG degradation, thereby greatly improving the tolerance; it also improves the selectivity for killer immune cells, thereby increasing the expression of PD-1 in immune cells in the microenvironment.

Nektar Therapeutics announced on NASDAQ on Oct. 7, 2015 that it had submitted an IND application for NKTR-214 to the U.S. Food and Drug Administration (FDA). The company had completed the preclinical studies of NKTR-214 and planned to initiate phase I and phase II clinical studies of the drug by the end of 2015.

In preclinical studies, by comparing the NKTR-214 with existing IL-2 treatment aldesleukin, NKTR-214 has an AUC in tumors nearly 400 times that of aldesleukin after a single dose. Furthermore, the data provided by Nektar indicated that NKTR-214 shows long-lasting immunotherapeutic effect in a number of preclinical animal models; in a tumor infiltration lymphocyte model, after treatment by NKTR-214, the ratio of CD8 positive T cells to CD4 positive T cells was about 450:1, demonstrating a potent killing effect on tumors. Therefore, NKTR-214 is an immunostimulatory factor anticancer drug having a therapeutic effect similar to that of an antibody, and can stimulate the growth of T cells, thereby realizing the killing effect on tumors. The results of preclinical study showed that the drug has long-acting anti-tumor therapeutic effect and is a promising anticancer drug.

However, in the actual production process, the NKTR-214 is formed by adding 6 PEGs on the normal IL-2 molecule by chemical modification, such that the IL-2 is selectively bound to the IL-2Rβγ receptor. In the present invention, the applicant provides a method for preparing a PEGylated biomolecule with controllable binding sites, which intervenes on the binding sites of the biomolecule *in vitro,* keeps the binding sites exposed and non-PEGylated, and leads to a stronger targeting capacity and a faster therapeutic effect of the biomolecule *in vivo.* Specifically, when IL-2 is modified, by regulating the binding site of IL-2, only 1 or 2 PEGs are added by PEGylation, such that the IL-2 can selectively bind to the IL-2Rβγ receptor.

### SUMMARY

The present invention is intended to provide a method for preparing a PEGylated biomolecule, and in particular to provide a method for preparing a PEGylated IL-2.

The invention is defined in claims 1-10.

The objectives of the present invention are implemented through the following technical schemes:
Provided is a method for preparing a PEGylated biomolecule, comprising:
(1) PEGylating a biomolecule;
(2) binding a barrier to at least one binding site in the PEGylated biomolecule;
(3) separating the PEGylated biomolecule not bound to the barrier; and
(4) separating the barrier and the PEGylated biomolecule bound thereto;
wherein the biomolecule in the step (1) is selected from biological macromolecules and biological micromolecules with specific binding effect to the barrier, and the biomolecule has at least one binding site for the barrier; the biomolecule is interleukin-2 and the barrier is an interleukin-2 receptor; specifically, the interleukin-2 receptor includes interleukin α, β and γ receptors.

The PEGylated biomolecule in the step (1) has the following structure:

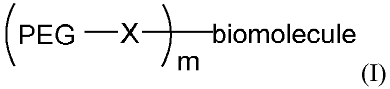

X is a linking group between the PEG and the biomolecule selected from: one or a combination of two or more of -(CH₂)a-, -(CR₁R₂)a-, -(CH₂)aNH-, -NHCO(CH₂)a-, -(CH₂)aCONH-, -(CH₂)aCO-, -CO(CH₂)a-, -(CH₂)aCONH(CH₂)a-, -(CH₂)a-S-S-(CH₂)a-, -(CH₂)aCOO(CH₂)a- and -(CH₂)a-S-(CH₂)a-.

Among them, a is an integer of 0-10. Preferably, a is an integer of 0-5, such as 0, 1, 2, 3, 4 or 5. More preferably, a is an integer of 0-3.

m is an integer of 1-12, specifically 1, 2, 3, 4, 5, 6, 7, 8, 9, 10, 11 or 12.

R₁ and R₂ are independently selected from: one or a combination of two or more of -H, a C₁₋₆ alkyl, -OR', -NHR', -N(R')₂, -CN, -F, -Cl, -Br, -I, -COR', -COOR', -OCOR', -CONHR' and -CON(R')₂. Preferably, R₁ and R₂ are independently selected from: one or a combination of two or more of -H, a C₁₋₃ alkyl (specifically, methyl, ethyl, *n*-propyl or isopropyl), -OH, a C₁₋₃ alkoxy, -NH₂, -F, -Cl, -Br and -I, and more preferably selected from: -H, -CH₃, -OH, -OCH₃ and -OCH₂CH₃. In one embodiment of the present invention, R₁ is -H, and R₂ is selected from: -CH₃, -OH, -OCH₃ and -OCH₂CH₃.

R' is selected from: -H, a C₁₋₆ alkyl, -F, -Cl, -Br and -I. Preferably, R' is selected from: -H and a C₁₋₃ alkyl, specifically methyl, ethyl, *n*-propyl or isopropyl.

Preferably, X is selected from: one or a combination of two or more of -CH₂-, -CH₂CH₂-, -CH₂CH₂CH₂-, -CH₂CONHCH₂-, -CH₂CONHCH₂CH₂-, -CH₂CONHCH₂CH₂NH-, -CH₂CH₂CONHCH₂-, -CH₂CO-, -CH₂CH₂CO-, -CH₂CH₂CONHCH₂CH₂-, -CH₂NH-, -CH₂CONH-, -COCH₂-, -COCH₂CH₂-, -COCH₂CH₂CH₂-, -CH₂-S-S-CH₂-, -CH₂COOCH₂- and -CH₂-S-CH₂-.

More preferably, X is selected from: -CH₂-, -CH₂CH₂-, -CH₂CH₂CONHCH₂-, -CH₂CH₂CO- and -CH₂COOCH₂-.

The PEG is a linear, Y-shaped or multi-branched polyethylene glycol residue including, for example, monomethoxy polyethylene glycol (mPEG), a linear double-ended PEG, a Y-shaped PEG, a 4-arm branched PEG, a 6-arm branched PEG and an 8-arm branched PEG.

Preferably, the PEG may have a molecular weight of 1-100 KDa, such as 1-10 KDa (specifically 1, 2, 3, 4, 5, 6, 7, 8, 9 or 10 KDa), 10-50 KDa (specifically 10, 15, 20, 25, 30, 35, 40, 45 or 50 KDa) or 50-100 KDa (specifically 50, 55, 60, 65, 70, 75, 80, 85, 90, 95 or 100 KDa); more preferably 10-50 KDa.

In one specific embodiment of the present invention, the PEG is a linear polyethylene glycol residue having a structure of general formula II or III: 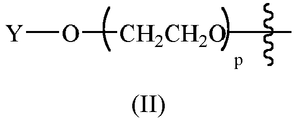 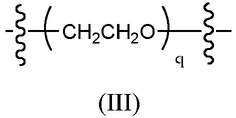
wherein p and q are integers independently selected from 1-2280, and preferably integers of 220-1140;
Y is a capping group, selected from: -H; a C₁₋₆ alkyl, specifically methyl, ethyl, *n*-propyl, isopropyl, *n*-butyl, isobutyl, *tert*-butyl, *n*-pentyl, *n*-hexyl or the like; a C₃₋₆ cycloalkyl, specifically cyclopropyl, cyclobutyl, cyclopentyl, cyclohexyl or the like; a substituted or unsubstituted C₆₋₁₀ aryl, specifically phenyl, naphthyl or the like; and -L-T;
L is a linking group between oxygen (O) and an end group T, selected from: one or a combination of two or more of -(CH₂)_{b}-, -(CR₃R₄)_{b}-, -(CH₂)_{b}NH-, -NHCO(CH₂)_{b}-, -(CH₂)_{b}CONH- and -CO(CH₂)_{b}-, wherein b is an integer of 0-10.
R₃ and R₄ are independently selected from: one or a combination of two or more of -H, a C₁₋₆ alkyl, -OR", -NHR", -N(R")₂, -CN, -F, -Cl, -Br, -I, -COR', -COOR", -OCOR", -CONHR" and -CON(R")₂.
R" is selected from: -H, a C₁₋₆ alkyl, -F, -Cl, -Br and -I.

Preferably, L is selected from: one or a combination of two or more of -CH₂-, -CH₂CH₂-, -CH₂CH₂CH₂-, -CONH-, -NH-, -CO-, -CONHCH₂-, -CH₂NH-, -CH₂CONH- and -COCH₂-.

T is an end group, selected from: -H; a C₁₋₆ alkyl; a C₃₋₆ cycloalkyl; a substituted or unsubstituted C₆₋₁₀ aryl; a residue of a monosaccharide, specifically glucose, fructose, galactose, ribose deoxyribose and the like; and a residue of an oligosaccharide, specifically disaccharide (sucrose, lactose and the like) and trisaccharide (gentiotriose, raffinose and the like).

Preferably, T is selected from: methyl, ethyl, isopropyl, cyclopropyl, cyclobutyl, cyclohexyl, benzyl,

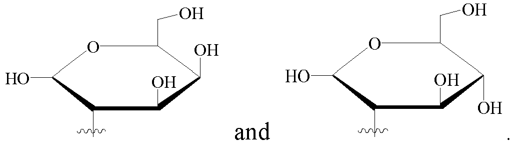

Preferably, Y is selected from: methyl, ethyl, isopropyl, cyclopropyl, cyclobutyl, cyclohexyl, benzyl,

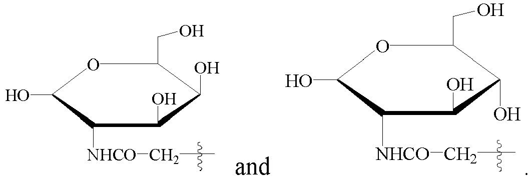

In one preferred embodiment of the present invention, Y is methyl.

In another preferred embodiment of the present invention, Y is 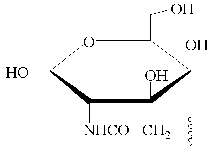 or

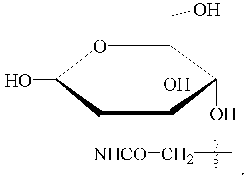

In one specific embodiment of the present invention, the PEG is a Y-shaped polyethylene glycol residue having a structure of general formula IV or V: 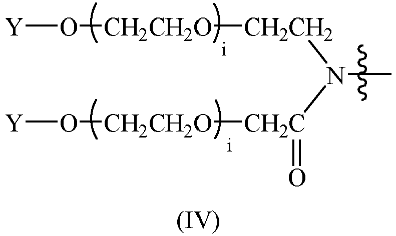 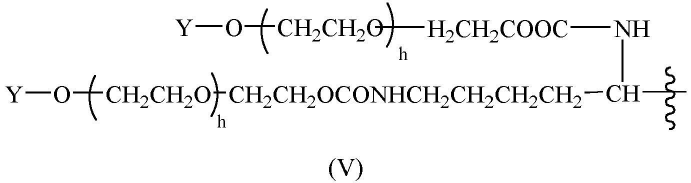 wherein i and h are integers independently selected from 1-1140, and preferably integers of 110-570.

Y has the same definition as described for general formula II above, and is preferably selected from: methyl, ethyl, isopropyl, cyclopropyl, cyclobutyl, cyclohexyl, benzyl, 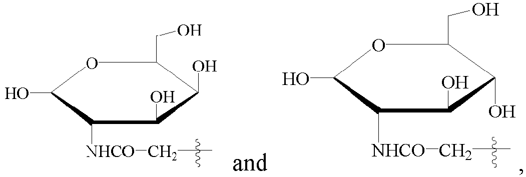 and more preferably selected from: methyl,

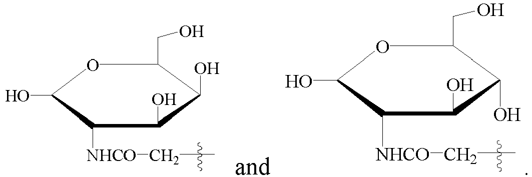

In one specific embodiment of the present invention, the PEG is a multi-branched polyethylene glycol residue having a structure of general formula VI: 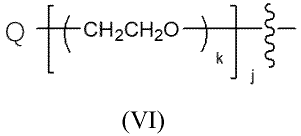
wherein k is an integer of 1-760, and preferably an integer of 70-380.
j is an integer of 3-8.
Q is a residue of a core molecule of a multi-branched polyethylene glycol selected from: pentaerythritol, oligomeric pentaerythritol, methyl glucoside, sucrose, diethylene glycol, propylene glycol, glycerol and polyglycerol; preferably, Q is selected from: pentaerythritol, dipentaerythritol and tripentaerythritol.

Preferably, the multi-branched polyethylene glycol residue has the following structure: 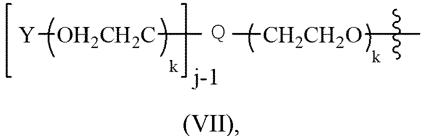 wherein Q, k and j have the same definitions as described for general formula VI above, and Y has the same definition as described for general formula II above, and is preferably selected from: methyl, ethyl, isopropyl, cyclopropyl, cyclobutyl, cyclohexyl, benzyl, 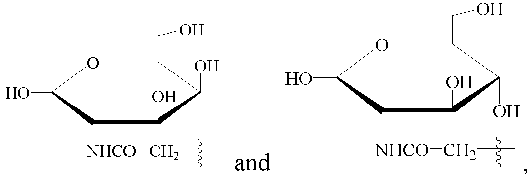 and more preferably selected from: methyl,

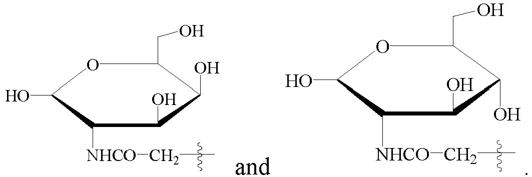

In one preferred embodiment of the present invention, the multi-branched polyethylene glycol residue has the following structure: 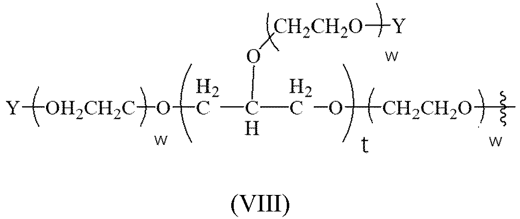
wherein w is an integer of 1-570, and preferably an integer of 55-285;
t is an integer of 1-10 (specifically 1, 2, 3, 4, 5, 6, 7, 8, 9 or 10), and preferably an integer of 1-6;
wherein Y has the same definition as described for general formula II above, and is preferably selected from: methyl, ethyl, isopropyl, cyclopropyl, cyclobutyl, cyclohexyl, benzyl, 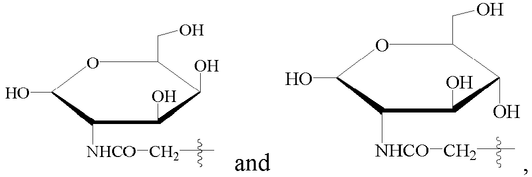 and more preferably selected from: methyl,

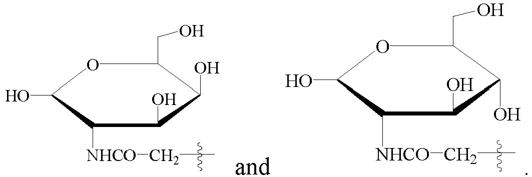

In another preferred embodiment of the present invention, the multi-branched polyethylene glycol residue has the following structure: 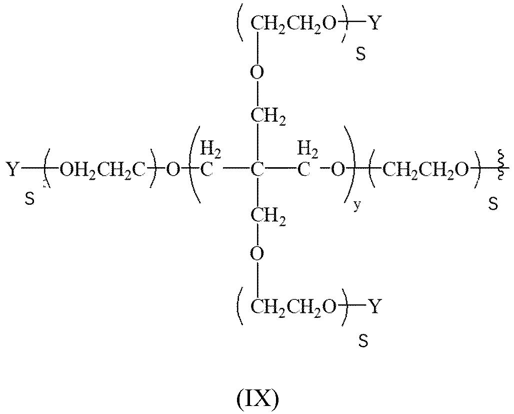
wherein s is an integer of 1-280, and preferably an integer of 28-140;
y is an integer of 1-10, specifically 1, 2, 3, 4, 5, 6, 7, 8, 9 or 10, preferably an integer of 1-5, and more preferably an integer of 1-3.
Y has the same definition as described for general formula II above, and is preferably selected from: methyl, ethyl, isopropyl, cyclopropyl, cyclobutyl, cyclohexyl, benzyl, 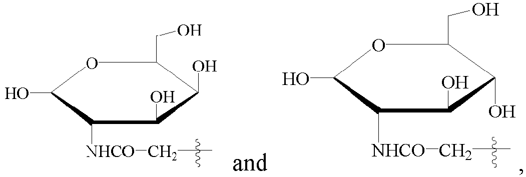 and more preferably selected from: methyl,

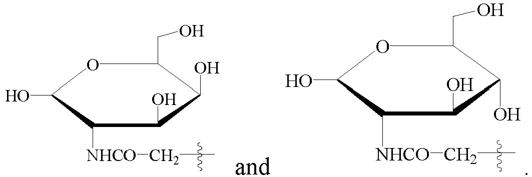

Preferably, the step (1) further comprises separating and purifying the PEGylated biomolecule. The separating and purifying are methods well known to those skilled in the art, such as membrane dialysis separation, gel filtration separation, or the like.

Separating the PEGylated biomolecule not bound to the barrier in the step (3) and separating the barrier and the PEGylated biomolecule in the step (4) include: one or a combination of two or more of centrifugal separation, precipitation separation, filtration separation, foam separation, extraction separation, membrane separation, chromatographic separation, electrophoretic separation and gradient elution.

Specifically, the present invention provides a method for preparing a PEGylated IL-2, comprising:
(1) PEGylating to couple PEG with IL-2;
(2) binding the PEGylated IL-2 to IL-2α receptor, IL-2β receptor and IL-2γ receptor;
(3) separating the PEGylated IL-2 not bound to the IL-2α receptor, the IL-2β receptor and the IL-2γ receptor; and
(4) separating the IL-2α receptor and the PEGylated IL-2 bound thereto.

Preferably, the step (1) further comprises separating and purifying the PEG-IL-2 conjugate.

Preferably, the present invention provides a method for preparing a PEGylated IL-2, comprising:
(1) preparing an IL-2α receptor affinity column;
(2) PEGylating to couple PEG with IL-2;
(3) separating and purifying the PEG-IL-2 conjugate in the step (2) to give the PEGylated IL-2;
(4) binding the PEGylated IL-2 to the IL-2α receptor, the IL-2β receptor and the IL-2γ receptor on the affinity column;
(5) separating the PEGylated IL-2 not bound to the IL-2α receptor, the IL-2β receptor and the IL-2γ receptor; and
(6) separating the IL-2α receptor and the PEGylated IL-2 bound thereto by gradient elution.
Unless defined otherwise, all technical and scientific terms used herein have the same meaning as commonly understood by those skilled in the art to which the present invention relates, such as the following terms.

The terms "interleukin-2", "interleukin 2" and "IL-2" used herein have the same meaning and can be used interchangeably to refer to a natural interleukin 2, a recombinant protein (e.g., recombinant human interleukin 2) or a mutant that has the function of natural IL-2 (e.g., "IL-2-C125A/L18M/L19S" as described in "Recombinant Human Interleukin-2 (IL-2) Mutant Cloning, Expression and Purification in Pichia pastoris System", Liu Yan, Ph.D. dissertation), including products obtained by tissue culture, protein synthesis and cell culture (natural, recombinant cells or mutant). Methods for extracting and separating the natural or recombinant IL-2, or mutants are well known to those skilled in the art.

As used herein, the term "pharmaceutically acceptable" refers to a substance that is physiologically compatible and do not cause gastrointestinal disorders, allergic reactions such as dizziness, or the like after administration to humans. The additive may be any of excipients, disintegrants, binders, lubricants, suspending agents, stabilizers, and the like. Examples of the excipients include lactose, mannitol, isomalt, microcrystalline cellulose, silicified microcrystalline cellulose, powdered cellulose, and the like. Examples of the disintegrants include low-substituted hydroxypropyl cellulose, crospovidone, sodium starch glycolate, croscarmellose sodium, starch, and the like. Examples of the binders include hydroxypropyl cellulose, hydroxypropyl methylcellulose, povidone, copovidone, pregelatinized starch, and the like. Examples of the lubricants include stearic acid, magnesium stearate, sodium fumaryl stearate, and the like; examples of the wetting agents include polyoxyethylene sorbitan fatty acid ester, poloxamer, polyoxyethylene castor oil derivatives, and the like. Examples of the suspending agents include hydroxypropyl methylcellulose, hydroxypropyl cellulose, povidone, copovidone, sodium carboxymethyl cellulose, methylcellulose, and the like. Examples of the stabilizers include citric acid, fumaric acid, succinic acid, and the like. In addition, the pharmaceutical composition disclosed herein may further include any of anticoagulants, flavor enhancers, emulsifiers, preservatives, and the like.

The pharmaceutical composition described herein may be a tablet (including dragee tablet, film coating tablet, sublingual tablet, oral disintegrating tablet, buccal tablet, etc.), a pill, a powder, a granule, a capsule (including soft capsule and microcapsule), a lozenge, a syrup, a liquid, an emulsion, a suspension, a controlled release formulation (e.g., instantaneous release formulation, sustained release formulation, sustained release microcapsule), an aerosol, a film (e.g., oral disintegrating film, oral mucosa-adherent film), an injection (e.g., subcutaneous injection, intravenous injection, intramuscular injection, intraperitoneal injection), an intravenous drip infusion, a transdermal formulation, an ointment, a lotion, an adhesive formulation, a suppository (e.g., rectal suppository, vaginal suppository), a pellet, a nasal formulation, a pulmonary formulation (inhalant), an eye drop, an oral or parenteral formulation (e.g., intravenous, intramuscular, subcutaneous, intraorgan, intranasal, intradermal, instillation, intracerebral and intrarectal administrations, administration to the vicinity of tumors, and direct administration to lesions), and the like. Preferably, the pharmaceutical composition is an injection.

The pharmaceutically acceptable excipients described herein are preferably pharmaceutically acceptable excipients for injection, such as isotonic sterile saline (sodium dihydrogen phosphate, disodium hydrogen phosphate, sodium chloride, potassium chloride, calcium chloride, magnesium chloride, etc., or mixtures of the above salts), or dry excipients, e.g., freeze-dried compositions which are properly formed into injectable solutes by addition of sterile water or physiological saline.

The PEGylated IL-2 of the present invention may be abbreviated as PEG-IL-2.

### BRIEF DESCRIPTION OF THE DRAWINGS

FIG. 1 is a gel chromatography separation pattern of the PEG-IL-2.
FIG. 2 shows the SDS-PAGE results of the PEG-IL-2, wherein 1 represents IL-2, 2 represents peak 1, 3 represents peak 2, and 4 represents a protein standard.

### DETAILED DESCRIPTION

The technical schemes in the examples of the present invention will be described clearly and completely below, and it is apparent that the examples described herein are only some examples of the present invention, but not all of them. Based on the examples of the present invention, all other examples obtained by those of ordinary skill in the art without creative work shall fall within the protection scope of the present invention.

### Example 1. Preparation of IL-2α receptor affinity column

S1: 0.8 g of CNBr-activated sehparose (sehparose CNBr) was added to 12 mL of 1 mM HCl, the system was manually and slowly mixed, and incubated in a refrigerator at 4 °C for reaction for 15 min;
S2: preparation of coupling buffer: 0.1 M NaHCO₃, pH 8.3 was mixed well with 0.5 M NaCl, and 12 mL of the coupling buffer was taken to wash the swollen filler in the previous step;
S3: 4 vials of IL-2α receptors with a total of 4 mg was taken and dissolved in 2 mL of coupling buffer, and the mixture was added to the swollen filler in the step S2;
S4: the mixture was slowly shaken at room temperature for 2 h;
S5: excess receptors were washed out with 5 column volumes of the coupling buffer;
S6: 0.1 M Tris-HCl, pH 8.0 was added, the mixture was let stand at room temperature for 2 h, and then Tris-HCl was discarded;
S7: the residue was washed with 5 column volumes of 0.1 M acetic acid/sodium acetate buffer (pH 4.0) containing 0.5 M NaCl and then washed with 5 column volumes of 0.1 M Tris-HCl buffer (pH 8.0) containing 0.5 M NaCl;
S8: the steps S6 and S7 were repeated 3 times;
S9: 0.5 mL of 0.1 M Tris-HCl, pH 8.0 was added;
S10: the sepharose resin was filled into a 5 mL empty column and then stored in a refrigerator at 4 °C.

### Example 2. Coupling of PEG and IL-2

4.9 mg of methoxy polyethylene glycol-succinimidyl propionate (M-SPA-20K) having a molecular weight of 20 KDa was dissolved in 24 µL of 2 mM HCl to prepare a 200 mg/mL solution. 4.7 µL of the solution was taken and added to 420 µL of a 1.1 mg/mL IL-2 solution, and 60 µL of 0.05 M sodium tetraborate, pH 9.77, was added. The mixture was let stand at room temperature for reaction for 1 h. After the reaction was completed, 40 µL of 2 M HAC was added to terminate the reaction to obtain a PEG-IL-2 conjugate.

### Example 3. Gel filtration separation of PEG-IL-2 conjugate

The gel filtration chromatography settings are as follows: mobile phase: 5 mM PBS, flow rate: 0.75 mL/min, column: superdex 200 increase 10/300GL.

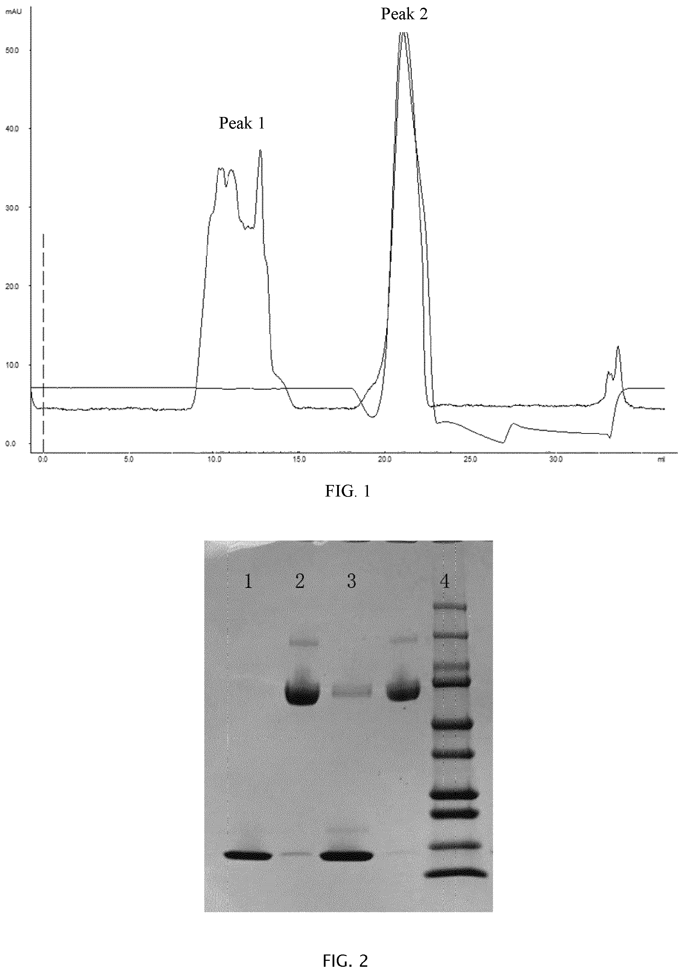

The separation procedures are as follows:
S1: the chromatographic system was equilibrated until the UV curve was flat;
S2: the elution peak was collected after the sample was loaded;
S3: the fractions was collected, concentrated by ultrafiltration and then detected by sodium dodecyl sulfate-polyacrylamide gel electrophoresis (SDS-PAGE). The results are shown in FIGs. 1 and 2.

Results: peak 1 in FIG. 1 was confirmed as PEGylated IL-2 (PEG-IL-2) by SDS-PAGE.

### Example 4. IL-2α receptor affinity column screening for PEGylated IL-2

S1: about 6 mL of the sample, PEGylated IL-2 (peak 1) prepared in Example 3 by filtration and separation, was injected 3 times;
S2: an IL-2α receptor affinity column containing 4 mg of receptors prepared in Example 1 was used;
S3: the mobile phases were prepared, phase A: 5 mM PBS and phase B: 0.2 M HAC + 0.2 M NaCl;
S4: the flow rate of the mobile phase was set as 0.45 mL/min;
S5: the column was equilibrated with phase A until the UV curve was flat;
S6: the PEGylated IL-2 was injected 3 times by the injection loop;
S7: the mixture was subjected to gradient elution, and the flow-through fraction and elution peak were collected after the sample was loaded.

### Example 5. Receptor affinity assay

The flow-through fraction and the elution fraction in Example 4 were collected, concentrated and subjected to affinity assay using an SPR system. The receptor proteins were IL-2α receptor and IL-2β receptor. The test results are shown in Table 1:

**Table 1. Results of affinity assay for flow-through fraction and elution fraction**

| Samples | Flow-through fraction | Elution fraction |
|---|---|---|
| IL-2/IL-2Rα (µM) | 15.4 | 1.89 |
| IL-2/IL-2Rβ (µM) | 124900 | 29.68 |

By analyzing the affinity assay results of the flow-through fraction and the elution fraction, we found that the flow-through fraction had an affinity for IL-2β receptor far greater than that for the IL-2α receptor, which indicates that the α receptor binding sites in the PEG-IL-2 in the flow-through fraction were occupied, leading to a stronger binding to the β receptor. Under the same test conditions, IL-2 had an affinity of 0.023 for the α receptor and an affinity of 2.80 for the β receptor. Compared with the receptor affinity of the elution fraction, the elution fraction had an 82-fold affinity for the α receptor of 1.89, and a 10-fold affinity for the β receptor of 29.68. It can be seen that the PEGylated IL-2 with controllable binding sites prepared by the methods according to Examples 1-4 has a significantly increased affinity for the IL-2α receptor. Finally, it should be noted that, the above examples are only used to illustrate the technical schemes of the present invention, but not to limit the present invention. Although the present invention is described in detail with reference to the examples described above, it will be understood by those skilled in the art that, the technical schemes in the examples described above can still be modified, or some or all of the technical features can be equivalently replaced; and these modifications or replacements do not make the technical schemes corresponding thereto depart from the scope of the technical solutions in the examples of the present invention.

## Claims

1. A method for preparing a PEGylated biomolecule, sequentially comprising the following steps:
(1) PEGylating a biomolecule;
(2) binding a barrier to at least one binding site in the PEGylated biomolecule;
(3) separating the PEGylated biomolecule not bound to the barrier; and
(4) separating the barrier and the PEGylated biomolecule bound thereto;
wherein the biomolecule in the step (1) is selected from biological macromolecules and biological micromolecules with specific binding effect to the barrier, and the biomolecule has at least one binding site for the barrier;
the barrier is an IL-2 receptor and the biomolecule is IL-2, and the IL-2 receptor includes IL-2α receptor, IL-2β receptor and IL-2γ receptor.

2. The method according to claim 1, wherein the step (1) further comprises separating and purifying the PEGylated biomolecule; and the separating in the step (3) and the step (4) includes: one or a combination of two or more of centrifugal separation, precipitation separation, filtration separation, foam separation, extraction separation, membrane separation, chromatographic separation, electrophoretic separation and gradient elution.

3. The method according to claim 1, wherein the PEGylated biomolecule in the step (1) has the following structure: 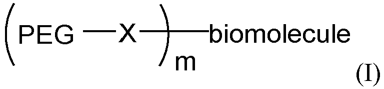
m is an integer of 1-12, specifically 1, 2, 3, 4, 5, 6, 7, 8, 9, 10, 11 or 12;
X is a linking group between the PEG and the biomolecule selected from: one or a combination of two or more of -(CH₂)a-, -(CR₁R₂)a-, -(CH₂)aNH-, -NHCO(CH₂)a-, -(CH₂)aCONH-, -(CH₂)aCO-, -CO(CH₂)a-, -(CH₂)aCONH(CH₂)a-, -(CH₂)a-S-S-(CH₂)a-, -(CH₂)aCOO(CH₂)a- and -(CH₂)a-S-(CH₂)a-;
a is an integer of 0-10;
R₁ and R₂ are independently selected from: one or a combination of two or more of -H, a C₁₋₆ alkyl, -OR', -NHR', -N(R')₂, -CN, -F, -Cl, -Br, -I, -COR', -COOR', -OCOR', -CONHR' and - CON(R')₂;
R' is selected from: -H, a C₁₋₆ alkyl, -F, -Cl, -Br and -I;
the PEG is a linear, Y-shaped or multi-branched polyethylene glycol residue including monomethoxy polyethylene glycol (mPEG), a linear double-ended PEG, a Y-shaped PEG, a 4-arm branched PEG, a 6-arm branched PEG and an 8-arm branched PEG with a molecular weight of 1-100 KDa.

4. The method according to claim 3, wherein a is an integer of 0-5;
R₁ and R₂ are independently selected from: one or a combination of two or more of -H, a C₁₋₃ alkyl, -OH, a C₁₋₃ alkoxy, -NH₂, -F, -Cl, -Br and -I;
R' is selected from: -H and a C₁₋₃ alkyl;
the PEG is a linear polyethylene glycol residue having a structure of general formula II or III: 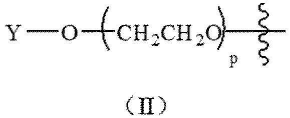 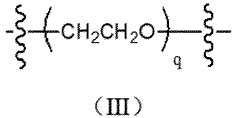 wherein p and q are independently selected from an integer of 1-2280;
the PEG is a Y-shaped polyethylene glycol residue having a structure of general formula IV or V: 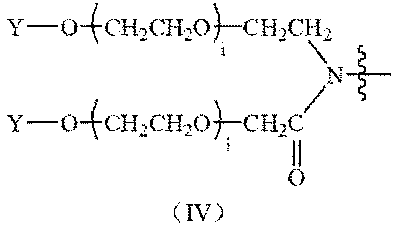 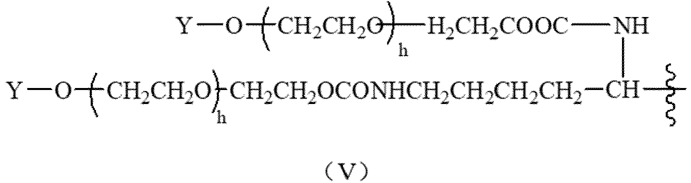 wherein i and h are independently selected from an integer of 1-1140;
or the PEG is a multi-branched polyethylene glycol residue having a structure of a general formula VI: 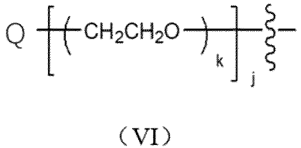 wherein k is an integer of 1-760, and j is an integer of 3-8;
Y is a capping group, selected from: -H; a C₁₋₆ alkyl, specifically methyl, ethyl, *n*-propyl, isopropyl, *n*-butyl, isobutyl, *tert-*butyl, *n*-pentyl or *n*-hexyl; a C₃₋₆ cycloalkyl, specifically cyclopropyl, cyclobutyl, cyclopentyl or cyclohexyl; a substituted or unsubstituted C₆₋₁₀ aryl, specifically phenyl or naphthyl; and -L-T; wherein L is a linking group between oxygen (O) and an end group T, selected from: one or a combination of two or more of -(CH₂)_{b}-, -(CR₃R₄)_{b}-, - (CH₂)_{b}NH-, -NHCO(CH₂)_{b}-, -(CH₂)_{b}CONH- and -CO(CH₂)_{b}-, wherein b is an integer of 0-10;
T is an end group, selected from: -H; a C₁₋₆ alkyl; a C₃₋₆ cycloalkyl; a substituted or unsubstituted C₆₋₁₀ aryl; a residue of a monosaccharide, specifically glucose, fructose, galactose, ribose and deoxyribose; and a residue of an oligosaccharide, specifically disaccharide (sucrose, lactose) and trisaccharide (gentiotriose, raffinose);
R₃ and R₄ are independently selected from: one or a combination of two or more of -H, a C₁₋₆ alkyl, -OR", -NHR", -N(R")₂, -CN, -F, -Cl, -Br, -I, -COR', -COOR", -OCOR", -CONHR" and - CON(R")₂;
R" is selected from: -H, a C₁₋₆ alkyl, -F, -Cl, -Br and -I;
Q is a residue of a core molecule of a multi-branched polyethylene glycol selected from: pentaerythritol, oligomeric pentaerythritol, methyl glucoside, sucrose, diethylene glycol, propylene glycol, glycerol and polyglycerol.

5. The method according to claim 4, wherein T is selected from: methyl, ethyl, isopropyl, cyclopropyl, cyclobutyl, cyclohexyl, benzyl, 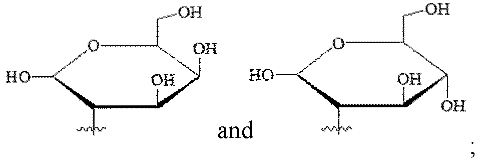
L is selected from: one or a combination of two or more of -CH₂-, -CH₂CH₂-, -CH₂CH₂CH₂-, - CONH-, -NH-, -CO-, -CONHCH₂-, -CH₂NH-, -CH₂CONH- and -COCH₂-;
Y is selected from: methyl, ethyl, isopropyl, cyclopropyl, cyclobutyl, cyclohexyl, benzyl, 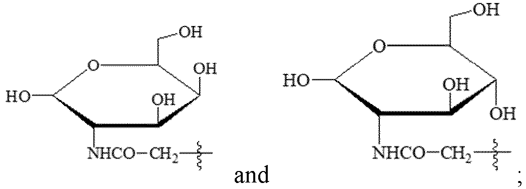
Q is selected from: pentaerythritol, dipentaerythritol and tripentaerythritol.

6. The method according to claim 4, wherein the multi-branched polyethylene glycol residue has the following structure: 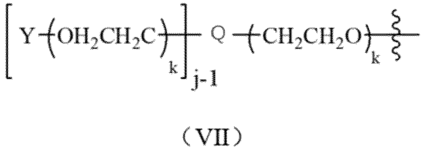 or the multi-branched polyethylene glycol residue has the following structure: 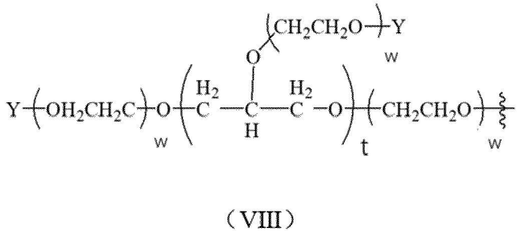 wherein w is an integer of 1-570, and t is an integer of 1-10;
or the multi-branched polyethylene glycol residue has the following structure: 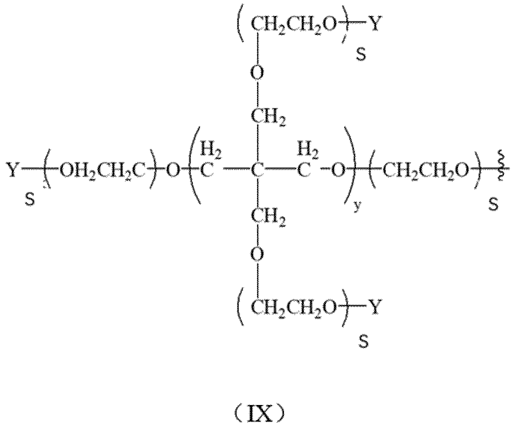 wherein s is an integer of 1-280, and y is an integer of 1-10.

7. The method according to any of claim 4, wherein a is an integer of 0-3; R₁ and R₂ are independently selected from: -H, -CH₃, -OH, -OCH₃ and -OCH₂CH₃.

8. The method according to any of claims 4-7, wherein X is selected from: one or a combination of two or more of -CH₂-, -CH₂CH₂-, -CH₂CH₂CH₂-, -CH₂CONHCH₂-, -CH₂CONHCH₂CH₂-, - CH₂CONHCH₂CH₂NH-, -CH₂CH₂CONHCH₂-, -CH₂CO-, -CH₂CH₂CO-, - CH₂CH₂CONHCH₂CH₂-, -CH₂NH-, -CH₂CONH-, -COCH₂-, -COCH₂CH₂-, -COCH₂CH₂CH₂-, - CH₂-S-S-CH₂-, -CH₂COOCH₂- and -CH₂-S-CH₂-.

9. The method according to any of claims 1-8, wherein the method is a method for preparing a PEGylated IL-2, sequentially comprising the following steps:
(1) PEGylating to couple PEG with IL-2;
(2) binding the PEGylated IL-2 to IL-2α receptor, IL-2β receptor and IL-2γ receptor;
(3) separating the PEGylated IL-2 not bound to the IL-2α receptor, the IL-2β receptor and the IL-2γ receptor; and
(4) separating the IL-2α receptor and the PEGylated IL-2 bound thereto; wherein, the step (1) further comprises separating and purifying the PEG-IL-2 conjugate.

10. The method according to claim 9, wherein the method for preparing the PEGylated IL-2 comprises:
(1) preparing an IL-2α receptor affinity column;
(2) PEGylating to couple PEG with IL-2;
(3) separating and purifying the PEG-IL-2 conjugate in the step (2) to give the PEGylated IL-2;
(4) binding the PEGylated IL-2 to the IL-2α receptor, the IL-2β receptor and the IL-2γ receptor on the affinity column;
(5) separating the PEGylated IL-2 not bound to the IL-2α receptor, the IL-2β receptor and the IL-2γ receptor; and
(6) separating the IL-2α receptor, the IL-2β receptor and the IL-2γ receptor and the PEGylated IL-2 bound thereto by gradient elution.

## Patentansprüche

1. Verfahren zur Herstellung eines PEGylierten Biomoleküls, sequentiell umfassend die folgenden Schritte:
(1) PEGylieren eines Biomoleküls;
(2) Binden einer Barriere an mindestens eine Bindungsstelle in dem PEGylierten Biomolekül;
(3) Trennen des PEGylierten Biomoleküls, das nicht an die Barriere gebunden ist; und
(4) Trennen der Barriere und des daran gebundenen PEGylierten Biomoleküls;
wobei das Biomolekül in Schritt (1) aus biologischen Makromolekülen und biologischen Mikromolekülen mit spezifischer Bindungswirkung an die Barriere ausgewählt ist und das Biomolekül mindestens eine Bindungsstelle für die Barriere aufweist;
die Barriere ein IL-2-Rezeptor ist und das Biomolekül IL-2 ist und der IL-2-Rezeptor einen IL-2α-Rezeptor, IL-2β-Rezeptor und IL-2γ-Rezeptor beinhaltet.

2. Verfahren nach Anspruch 1, wobei Schritt (1) ferner Trennen und Aufreinigen des PEGylierten Biomoleküls umfasst; und das Trennen in Schritt (3) und Schritt (4) Folgendes beinhaltet: eines oder eine Kombination aus zwei oder mehreren von einer Zentrifugaltrennung, Fällungstrennung, Filtrationstrennung, Schaumtrennung, Extraktionstrennung, Membrantrennung, chromatographischen Trennung, elektrophoretischen Trennung und Gradientenelution.

3. Verfahren nach Anspruch 1, wobei das PEGylierten Biomolekül in Schritt (1) die folgende Struktur aufweist: 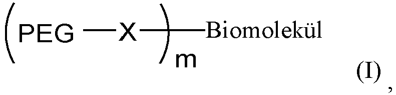
m eine ganze Zahl von 1-12 ist, insbesondere 1, 2, 3, 4, 5, 6, 7, 8, 9, 10, 11 oder 12;
X eine Verbindungsgruppe zwischen dem PEG und dem Biomolekül ist, die aus Folgendem ausgewählt ist: einem oder einer Kombination aus zwei oder mehr von -(CH₂)a-, - (CR₁R₂)a-, -(CH₂)aNH-, -NHCO(CH₂)a-, -(CH₂)aCONH-, -(CH₂)aCO-, -CO(CH₂)a-, - (CH₂)aCONH(CH₂)a-, -(CH₂)a-S-S-(CH₂)a-, -(CH₂)aCOO(CH₂)a- und -(CH₂)a-S-(CH₂)a-;
a eine ganze Zahl von 0-10 ist;
R₁ und R₂ unabhängig aus Folgendem ausgewählt sind: einem oder einer Kombination aus zwei oder mehr von -H, einem C₁₋₆-Alkyl, -OR', -NHR', -N(R')₂, -CN, -F, -Cl, -Br, -I, -COR', - COOR', -OCOR', -CONHR' und -CON(R')₂;
R' aus Folgendem ausgewählt ist: -H, einem C₁₋₆-Alkyl, -F, -Cl, -Br und -I;
das PEG ein linearer, Y-förmiger oder mehrfach verzweigter Polyethylenglycolrest ist, der Monomethoxypolyethylenglycol (mPEG), ein lineares doppelendiges PEG, ein Y-förmiges PEG, ein 4-armiges verzweigtes PEG, ein 6-armiges verzweigtes PEG und ein 8-armiges verzweigtes PEG mit einem Molekulargewicht von 1-100 KDa beinhaltet.

4. Verfahren nach Anspruch 3, wobei a eine ganze Zahl von 0-5 ist;
R₁ und R₂ unabhängig aus Folgendem ausgewählt sind: einem oder einer Kombination aus zwei oder mehr von -H, einem C₁₋₃-Alkyl, -OH, einem C₁₋₃-Alkoxy, -NH₂, -F, -Cl, -Br und -I;
R' aus Folgendem ausgewählt ist: -H und einem C₁₋₃-Alkyl;
das PEG ein linearer Polyethylenglycolrest mit einer Struktur der allgemeinen Formel II oder III ist: 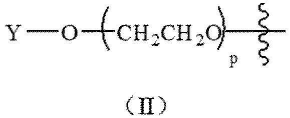 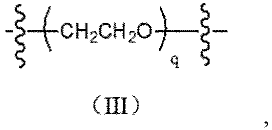 wobei p und q unabhängig voneinander aus einer ganzen Zahl von 1-2280 ausgewählt sind;
das PEG ein Y-förmiger Polyethylenglykolrest mit einer Struktur der allgemeinen Formel IV oder V ist: 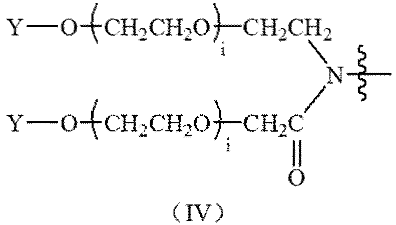 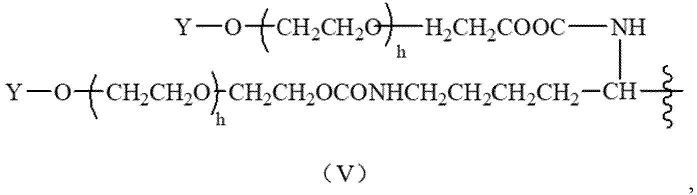
wobei i und h unabhängig aus einer ganzen Zahl von 1-1140 ausgewählt sind;
oder das PEG ein mehrfach verzweigter Polyethylenglycolrest mit einer Struktur der allgemeinen Formel VI ist: 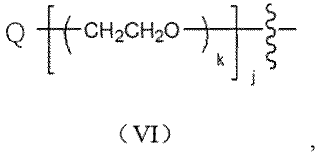 wobei k eine ganze Zahl von 1-760 ist und j eine ganze Zahl von 3-8 ist;
Y eine Verkappungsgruppe ist, die aus Folgendem ausgewählt ist: -H; einem C₁₋₆-Alkyl, insbesondere Methyl, Ethyl, *n*-Propyl, Isopropyl, *n*-Butyl, Isobutyl, *tert*-Butyl, *n*-Pentyl oder *n-*Hexyl; einem C₃₋₆ -Cycloalkyl, insbesondere Cyclopropyl, Cyclobutyl, Cyclopentyl oder Cyclohexyl; einem substituierten oder unsubstituierten C₆₋₁₀-Aryl, insbesondere Phenyl oder Naphthyl; und -L-T; wobei L eine Verbindungsgruppe zwischen Sauerstoff (O) und einer Endgruppe T ist, die aus Folgendem ausgewählt ist: einem oder einer Kombination aus zwei oder mehr von-(CH₂)₆-, -(CR₃R₄)_{b}-, -(CH₂)_{b}NH-, -NHCO(CH₂)_{b}-, -(CH₂)_{b}CONH- und -CO(CH₂)_{b}-, wobei b eine ganze Zahl von 0-10 ist;
T eine Endgruppe ist, die aus Folgendem ausgewählt ist: -H; einem C₁₋₆-Alkyl; einem C₃₋₆-Cycloalkyl; einem substituierten oder unsubstituierten C₆₋₁₀-Aryl, einem Rest eines Monosaccharids, insbesondere Glucose, Fructose, Galactose, Ribose und Desoxyribose; und einem Rest eines Oligosaccharids, insbesondere Disaccharid (Saccharose, Lactose) und Trisaccharid (Gentiotriose, Raffinose);
R₃ und R₄ unabhängig aus Folgendem ausgewählt sind: einem oder einer Kombination aus zwei oder mehr von -H, a C₁₋₆-Alkyl, -OR", -NHR", -N(R")₂, -CN, -F, -Cl, -Br, -I, -COR', -COOR", -OCOR", -CONHR" und - CON(R")₂;
R" aus Folgendem ausgewählt ist: -H, einem C₁₋₆-Alkyl, -F, -Cl, -Br und -I;
Q ein Rest eines Kernmoleküls eines mehrfach verzweigten Polyethylenglycols ist, das aus Folgendem ausgewählt ist: Pentaerythrit, oligomerem Pentaerythrit, Methylglucosid, Saccharose, Diethylenglycol, Propylenglycol, Glycerin und Polyglycerin.

5. Verfahren nach Anspruch 4, wobei T aus Folgendem ausgewählt ist: Methyl, Ethyl, Isopropyl, Cyclopropyl, Cyclobutyl, Cyclohexyl, Benzyl, 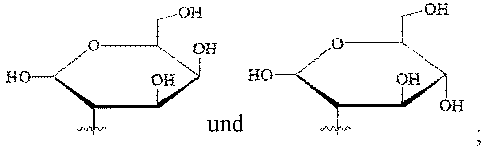
L aus Folgendem ausgewählt ist: einem oder einer Kombination aus zwei oder mehr von - CH₂-, -CH₂CH₂-, -CH₂CH₂CH₂-, -CONH-, -NH-, -CO-, -CONHCH₂-, -CH₂NH-, -CH₂CONH- und -COCH₂-,
Y aus Folgendem ausgewählt ist: Methyl, Ethyl, Isopropyl, Cyclopropyl, Cyclobutyl, Cyclohexyl, Benzyl 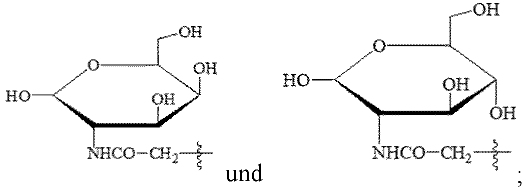
Q aus Folgendem ausgewählt ist: Pentaerythrit, Dipentaerythrit und Tripentaerythrit.

6. Verfahren nach Anspruch 4, wobei der mehrfach verzweigte Polyethylenglycolrest die folgende Struktur aufweist: 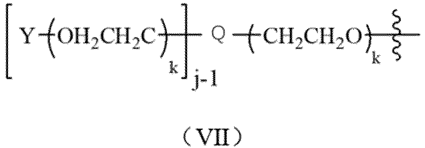 oder der mehrfach verzweigte Polyethylenglykolrest die folgende Struktur aufweist: 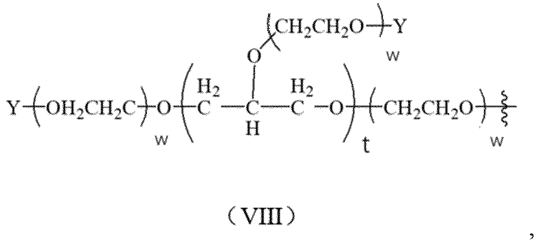 wobei w eine ganze Zahl von 1-570 ist und t eine ganze Zahl von 1-10 ist;
oder der mehrfach verzweigte Polyethylenglykolrest die folgende Struktur aufweist: 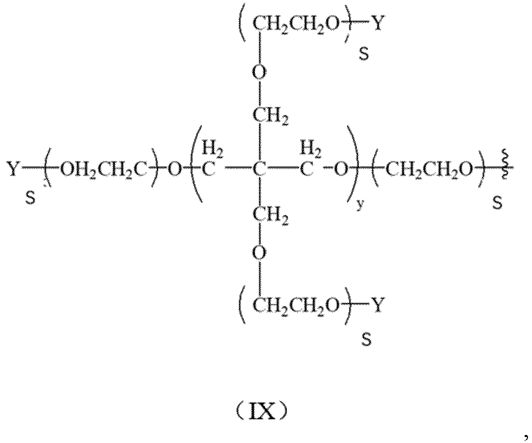 wobei s eine ganze Zahl von 1-280 ist und y eine ganze Zahl von 1-10 ist.

7. Verfahren nach einem der Ansprüche 4, wobei a eine ganze Zahl von 0-3 ist; R₁ und R₂ unabhängig aus Folgendem ausgewählt sind: -H, -CH₃, -OH, -OCH₃ und -OCH₂CH₃.

8. Verfahren nach einem der Ansprüche 4-7, wobei X aus Folgendem ausgewählt ist: einem oder einer Kombination aus zwei oder mehr von -CH₂-, -CH₂CH₂-, -CH₂CH₂CH₂-, -CH₂ CONHCH₂-, -CH₂CONHCH₂CH₂-, -CH₂CONHCH₂CH₂NH-, -CH₂CH₂CONHCH₂-, -CH₂CO-, - CH₂CH₂CO-, -CH₂CH₂CONHCH₂CH₂-, -CH₂NH-, -CH₂CONH-, -COCH₂-, -COCH₂CH₂-, - COCH₂CH₂CH₂-, -CH₂-S-S-CH₂-, -CH₂COOCH₂- und -CH₂-S-CH₂-.

9. Verfahren nach einem der Ansprüche 1-8, wobei das Verfahren ein Verfahren zur Herstellung eines PEGylierten IL-2 ist, sequentiell umfassend die folgenden Schritte:
(1) PEGylieren, um PEG mit IL-2 zu koppeln;
(2) Binden des PEGylierten IL-2 an einen IL-2α-Rezeptor, IL-2β-Rezeptor und IL-2γ-Rezeptor;
(3) Trennen des PEGylierten IL-2, das nicht an den IL-2α-Rezeptor, den IL-2β-Rezeptor und den IL-2γ-Rezeptor gebunden ist; und
(4) Trennen des IL-2α-Rezeptors und des daran gebundenen PEGylierten IL-2; wobei Schritt (1) ferner Trennen und Aufreinigen des PEG-IL-2-Konjugats umfasst.

10. Verfahren nach Anspruch 9, wobei das Verfahren zur Herstellung des PEGylierten IL-2 Folgendes umfasst:
(1) Herstellen einer IL-2α-Rezeptor-Affinitätssäule;
(2) PEGylieren, um PEG mit IL-2 zu koppeln;
(3) Trennen und Aufreinigen des PEG-IL-2-Konjugats in Schritt (2), um das PEGylierte IL-2 zu erhalten;
(4) Binden des PEGylierten IL-2 an den IL-2α-Rezeptor, den IL-2β-Rezeptor und den IL-2γ-Rezeptor an der Affinitätssäule;
(5) Trennen des PEGylierten IL-2, das nicht an den IL-2α-Rezeptor, den IL-2β-Rezeptor und den IL-2γ-Rezeptor gebunden ist; und
(6) Trennen des IL-2α-Rezeptors, des IL-2β-Rezeptors und des IL-2γ-Rezeptors und des daran gebundenen PEGylierten IL-2 durch Gradientenelution.

## Revendications

1. Procédé permettant la préparation d'une biomolécule PEGylée, comprenant séquentiellement les étapes suivantes :
(1) PEGylation d'une biomolécule ;
(2) liaison d'une barrière à au moins un site de liaison dans la biomolécule PEGylée ;
(3) séparation de la biomolécule PEGylée non liée à la barrière ; et
(4) séparation de la barrière et de la biomolécule PEGylée qui lui est liée ;
dans lequel la biomolécule dans l'étape (1) est choisie parmi les macromolécules biologiques et les micromolécules biologiques ayant un effet de liaison spécifique à la barrière, et la biomolécule a au moins un site de liaison pour la barrière ;
la barrière est un récepteur de l'IL-2 et la biomolécule est l'IL-2, et le récepteur de l'IL-2 comprend le récepteur de l'IL-2α, le récepteur de l'IL-2β et le récepteur de l'IL-2γ.

2. Procédé selon la revendication 1, dans lequel l'étape (1) comprend en outre la séparation et la purification de la biomolécule PEGylée ; et la séparation dans l'étape (3) et l'étape (4) comprend : une ou une combinaison de deux ou davantage parmi la séparation centrifuge, la séparation par précipitation, la séparation par filtration, la séparation par mousse, la séparation par extraction, la séparation par membrane, la séparation chromatographique, la séparation électrophorétique et l'élution par gradient.

3. Procédé selon la revendication 1, dans lequel la biomolécule PEGylée de l'étape (1) présente la structure suivante : 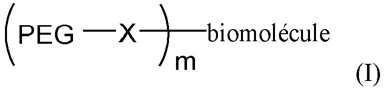
m est un nombre entier de 1 à 12, en particulier 1, 2, 3, 4, 5, 6, 7, 8, 9, 10, 11 ou 12 ;
X est un groupe de liaison entre le PEG et la biomolécule choisie parmi : un ou une combinaison de deux ou davantage des éléments suivants : -(CH₂)a-, -(CR₁R₂)a-, -(CH₂)aNH-, - NHCO(CH₂)a-, -(CH₂)aCONH-, -(CH₂)aCO-, -CO(CH₂)a-, -(CH₂)aCONH(CH₂)a-, -(CH₂)a-S-S-(CH₂)a-, -(CH₂)aCOO(CH₂)a- et -(CH₂)a-S-(CH₂)a- ;
a est un nombre entier de 0 à 10 ;
R₁ et R₂ sont indépendamment sélectionnés parmi : un ou une combinaison de deux ou davantage parmi -H, un alkyle en C₁₋₆, -OR', -NHR', -N(R')₂, -CN, -F, -Cl, -Br, -I, -COR', -COOR', -OCOR', -CONHR' et - CON(R')₂ ;
R' est choisi parmi : -H, un alkyle en C₁₋₆, -F, -Cl, -Br et -I ;
le PEG est un résidu de polyéthylène glycol linéaire, en forme de Y ou ramifié comprenant du monométhoxy polyéthylène glycol (mPEG), un PEG linéaire à deux extrémités, un PEG en forme de Y, un PEG ramifié à 4 branches, un PEG ramifié à 6 branches et un PEG ramifié à 8 branches ayant un poids moléculaire de 1 à 100 KDa.

4. Procédé selon la revendication 3, a étant un entier entre 0 et 5 ;
R₁ et R₂ sont indépendamment sélectionnés parmi : un ou une combinaison de deux ou davantage parmi -H, un alkyle en C₁₋₃, -OH, un alkoxy en C₁₋₃, -NH₂, -F, -Cl, -Br et -I ;
R' est choisi entre H et un alkyle en C₁₋₃ ;
le PEG est un résidu de polyéthylène glycol linéaire ayant une structure de formule générale II ou III : 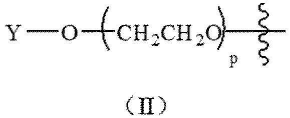 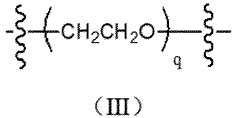 dans laquelle p et q sont indépendamment choisis parmi les nombres entiers de 1 à 2280 ;
le PEG est un résidu de polyéthylène glycol en forme de Y ayant une structure de formule générale IV ou V : 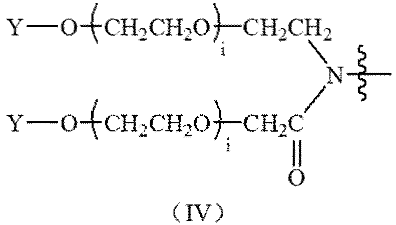 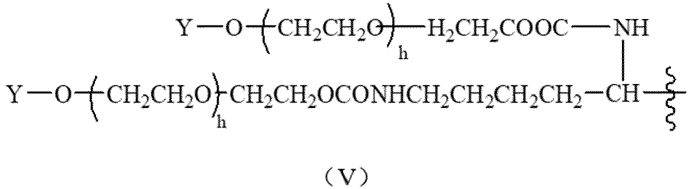 dans laquelle i et h sont choisis indépendamment parmi les nombres entiers de 1 à 1140 ;
ou le PEG est un résidu de polyéthylène glycol ramifié ayant une structure de formule générale VI : 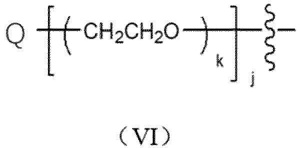 dans laquelle k est un nombre entier de 1 à 760, et j est un nombre entier de 3 à 8 ;
Y est un groupe de coiffage, choisi parmi : -H ; un C₁₋₆ alkyle, en particulier méthyle, éthyle, *n*-propyle, isopropyle, *n*-butyle, isobutyle, *tert*-butyle, *n*-pentyle ou *n*-hexyle ; un C₃₋₆ cycloalkyle, en particulier cyclopropyle, cyclobutyle, cyclopentyle ou cyclohexyle ; un C₆₋₁₀ aryle substitué ou non substitué, en particulier phényle ou naphtyle ; et -L-T ; où L est un groupe de liaison entre l'oxygène (O) et un groupe terminal T, choisi parmi : un ou une combinaison de deux ou davantage de -(CH₂)_{b}-, -(CR₃R₄)_{b}-, -(CH₂)_{b}NH-, -NHCO(CH₂)_{b}-, -(CH₂)_{b}CONH- et - CO(CH₂)_{b}-, où b est un nombre entier de 0 à 10 ;
T est un groupe terminal, choisi parmi : -H ; un alkyle en C₁₋₆ ; un cycloalkyle en C₃₋₆ ; un aryle en C₆₋₁₀ substitué ou non substitué ; un résidu d'un monosaccharide, en particulier le glucose, le fructose, le galactose, le ribose et le désoxyribose ; et un résidu d'un oligosaccharide, en particulier le disaccharide (saccharose, lactose) et le trisaccharide (gentiotriose, raffinose) ;
R₃ et R₄ sont indépendamment sélectionnés parmi : un ou une combinaison de deux ou davantage parmi -H, un alkyle en C₁₋₆, -OR", -NHR", -N(R")₂, -CN, -F, -Cl, -Br, -I, -COR', - COOR", -OCOR", -CONHR" et -CON(R")₂ ;
R" est choisi parmi : -H, un alkyle en C₁₋₆, -F, -Cl, -Br et -I ;
Q est un résidu d'une molécule de base d'un polyéthylène glycol ramifié choisi parmi : le pentaérythritol, le pentaérythritol oligomère, le méthyl glucoside, le saccharose, le diéthylène glycol, le propylène glycol, le glycérol et le polyglycérol.

5. Procédé selon la revendication 4, dans lequel T est choisi parmi : les groupes méthyle, éthyle, isopropyle, cyclopropyle, cyclobutyle, cyclohexyle, benzyle, 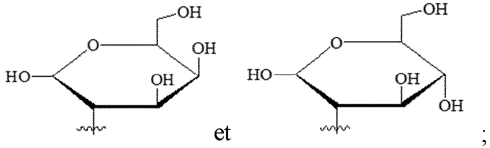
L est sélectionné parmi : un ou une combinaison de deux ou davantage parmi -CH₂-, - CH₂CH₂-, -CH₂CH₂CH₂-, -CONH-, -NH-, -CO-, -CONHCH₂-, -CH₂NH-, -CH₂CONH- et - COCH₂- ;
Y est choisi parmi : les groupes méthyle, éthyle, isopropyle, cyclopropyle, cyclobutyle, cyclohexyle, benzyle, 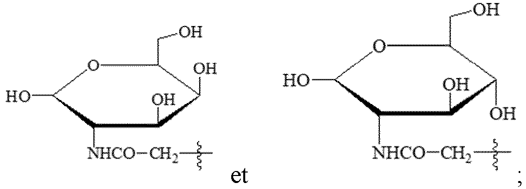
Q est choisi parmi : pentaérythritol, dipentaérythritol et tripentaérythritol.

6. Procédé selon la revendication 4, ledit résidu de polyéthylène glycol ramifié ayant la structure suivante : 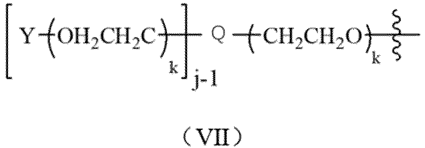 ou le résidu de polyéthylène glycol ramifié ayant la structure suivante : 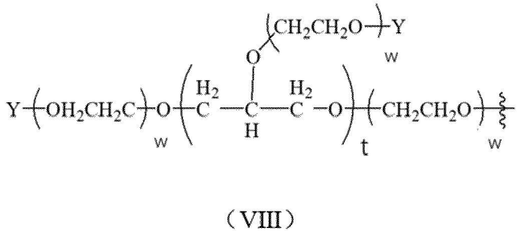 dans laquelle w est un nombre entier de 1 à 570, et t est un nombre entier de 1 à 10 ;
ou le résidu de polyéthylène glycol ramifié a la structure suivante : 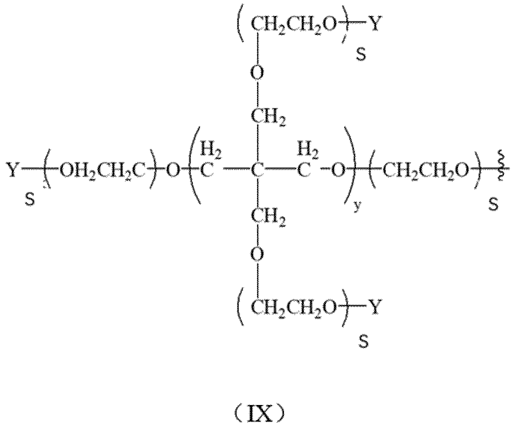 où s est un entier de 1 à 280, et y est un entier de 1 à 10.

7. Procédé selon la revendication 4, dans lequel a est un nombre entier de 0 à 3 ; R₁ et R₂ sont choisis indépendamment parmi : -H, -CH₃, -OH, -OCH₃ et -OCH₂CH₃.

8. Procédé selon l'une quelconque des revendications 4 à 7, dans lequel X est choisi parmi : un ou une combinaison de deux ou davantage parmi -CH₂-, -CH₂CH₂-, -CH₂CH₂CH₂-, - CH₂CONHCH₂-, -CH₂CONHCH₂CH₂-, -CH₂CONHCH₂CH₂NH-, -CH₂CH₂CONHCH₂-, - CH₂CO-, -CH₂CH₂CO-, -CH₂CH₂CONHCH₂CH₂-, -CH₂NH-, -CH₂CONH-, -COCH₂-, - COCH₂CH₂-, -COCH₂CH₂CH₂-, -CH₂-S-S-CH₂-, -CH₂COOCH₂- et -CH₂-S-CH₂-.

9. Procédé selon l'une quelconque des revendications 1 à 8, ledit procédé étant un procédé de préparation d'une IL-2 PEGylée, comprenant séquentiellement les étapes suivantes :
(1) PEGylation pour coupler PEG avec IL-2 ;
(2) liaison de l'IL-2 PEGylée au récepteur de l'IL-2α, au récepteur de l'IL-2β et au récepteur de l'IL-2γ ;
(3) séparation de l'IL-2 PEGylée non liée au récepteur de l'IL-2α, au récepteur de l'IL-2β et au récepteur de l'IL-2γ ; et
(4) séparation du récepteur de l'IL-2α et de l'IL-2 PEGylée qui lui est liée ; dans lequel l'étape (1) comprend en outre la séparation et la purification du conjugué PEG-IL-2.

10. Procédé selon la revendication 9, ledit procédé permettant la préparation de l'IL-2 PEGylée comprenant :
(1) préparation d'une colonne d'affinité du récepteur IL-2α ;
(2) PEGylation pour coupler PEG avec IL-2 ;
(3) séparation et purification du conjugué PEG-IL-2 à l'étape (2) pour donner l'IL-2 PEGylée ;
(4) liaison de l'IL-2 PEGylée au récepteur de l'IL-2α, au récepteur de l'IL-2β et au récepteur de l'IL-2γ sur la colonne d'affinité ;
(5) séparation de l'IL-2 PEGylée non liée au récepteur de l'IL-2α, au récepteur de l'IL-2β et au récepteur de l'IL-2γ ; et
(6) séparation du récepteur de l'IL-2α, du récepteur de l'IL-2β et du récepteur de l'IL-2γ et de l'IL-2 PEGylée qui leur est liée par élution en gradient.
